(19) Europäisches Patentamt
European Patent Office
Office européen des brevets

(11) **EP 4 317 460 B1**

(12) **EUROPEAN PATENT SPECIFICATION**

(45) Date of publication and mention
of the grant of the patent:
**31.12.2025 Bulletin 2026/01**

(21) Application number: **22775825.7**

(22) Date of filing: **25.03.2022**

(51) International Patent Classification (IPC):
$C12Q\ 1/70$ (2006.01)   $B01D\ 65/10$ (2006.01)
$B01D\ 69/02$ (2006.01)   $C12Q\ 1/22$ (2006.01)

(52) Cooperative Patent Classification (CPC):
**C12Q 1/22; B01D 65/10; B01D 69/02;**
C12N 2750/14321

(86) International application number:
**PCT/JP2022/014401**

(87) International publication number:
**WO 2022/203044 (29.09.2022 Gazette 2022/39)**

(54) **VIRAL CLEARANCE TEST METHOD**

TESTVERFAHREN FÜR VIRALE CLEARANCE

PROCÉDÉ DE TEST DE CLAIRANCE VIRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2021 JP 2021053527**

(43) Date of publication of application:
**07.02.2024 Bulletin 2024/06**

(73) Proprietor: **Asahi Kasei Life Science Corporation Tokyo 100-0006 (JP)**

(72) Inventors:
• **SHIRATAKI, Hironobu**
**Tokyo 100-0006 (JP)**
• **EBNER, Juergen**
**1220 Vienna (AT)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

(56) References cited:
**WO-A1-2018/075716    WO-A1-2022/072899**

• **HERBERT LUTZ ET AL: "Qualification of a novel inline spiking method for virus filter validation", BIOTECHNOLOGY PROGRESS, AMERICAN CHEMICAL SOCIETY, HOBOKEN, USA, vol. 27, no. 1, 27 September 2010 (2010-09-27), pages 121 - 128, XP072299057, ISSN: 8756-7938, DOI: 10.1002/BTPR.500**
• **BOHONAK DAVID M. ET AL: "Adapting virus filtration to enable intensified and continuous monoclonal antibody processing", vol. 37, no. 2, 11 December 2020 (2020-12-11), Hoboken, USA, XP055969924, ISSN: 8756-7938, Retrieved from the Internet <URL:https://onlinelibrary.wiley.com/doi/full-xml/10.1002/btpr.3088> DOI: 10.1002/btpr.3088**
• **YING LI ET AL: "Novel spiking methods developed for anion exchange chromatography operating in a continuous process", BIOTECHNOLOGY AND BIOENGINEERING, JOHN WILEY, HOBOKEN, USA, vol. 117, no. 11, 30 July 2020 (2020-07-30), pages 3379 - 3389, XP071052122, ISSN: 0006-3592, DOI: 10.1002/BIT.27500**

- ANONYMOUS: "Viral Safety - Practical Solutions for Risk Control", 1 June 2018 (2018-06-01), pages 1 - 39, XP055713559, Retrieved from the Internet <URL:https://www.pall.com/content/dam/pall/biopharm/lit-library/gated/special/19-07511-USD3364-Virus-Safety-Practical-Solutions-Risk-Control-BOOK-EN.PDF> [retrieved on 20200710]
- BIRTE KLEINDIENST, ANIKA MANZKE, PETER KOSIOL: "Continuous Processing: Challenges and Opportunities of Virus Filtration", PHARMACEUTICAL TECHNOLOGY, vol. 43, no. 1, 2 January 2019 (2019-01-02), pages 38 - 40, XP002805245
- YOSHIMOTO NORIKO, ICHIHARA TAKAMITSU, YAMAMOTO SHUICHI: "Connected flow-through chromatography processes as continuous downstream processing of proteins", MATEC WEB OF CONFERENCES, vol. 268, 1 January 2019 (2019-01-01), pages 01003, XP055969912, DOI: 10.1051/matecconf/201926801003
- SARAH A. JOHNSON; MATTHEW R. BROWN; SCOTT C. LUTE; KURT A. BRORSON: "Adapting viral safety assurance strategies to continuous processing of biological products", BIOTECHNOLOGY AND BIOENGINEERING, JOHN WILEY, HOBOKEN, USA, vol. 114, no. 6, 20 April 2017 (2017-04-20), Hoboken, USA, pages 1362 - 1362, XP071153347, ISSN: 0006-3592, DOI: 10.1002/bit.26245
- BOHONAK DAVID M., MEHTA USHMA, WEISS ERIC R., VOYTA GREG: "Adapting virus filtration to enable intensified and continuous monoclonal antibody processing", BIOTECHNOLOGY PROGRESS, AMERICAN CHEMICAL SOCIETY, vol. 37, no. 2, 1 March 2021 (2021-03-01), XP055969924, ISSN: 8756-7938, DOI: 10.1002/btpr.3088
- SHIRATAKI HIRONOBU, YOKOYAMA YOSHIRO, TANIGUCHI HIROKI, AZEYANAGI MIKU: "Analysis of filtration behavior using integrated column chromatography followed by virus filtration", BIOTECHNOLOGY AND BIOENGINEERING, JOHN WILEY, HOBOKEN, USA, vol. 118, no. 9, 1 September 2021 (2021-09-01), Hoboken, USA, pages 3569 - 3580, XP055969931, ISSN: 0006-3592, DOI: 10.1002/bit.27840

## Description

## Technical Field

The present invention relates to a viral clearance test method.

## Background Art

Biological material-containing therapeutic drug products include plasma fractionated therapeutic drug products obtained by purifying the blood and biotherapeutic drug products produced by biotechnology. These biological material-containing therapeutic drug products may have a risk of containing a virus. In general, biological material-containing therapeutic drug products should be ensured to be safe against a virus. The production of a biological material-containing therapeutic drug product, therefore, is required to have a step of sufficiently removing or inactivating a virus which is or may be contained in the biological material-containing therapeutic drug product, that is, a virus removal step (refer to, for example, Non-Patent Document 1). A test performed in the virus removal step to determine the virus removal or inactivation capacity is called "viral clearance test".

What is called "the most robust virus removal step" among the steps of producing a biological material-containing therapeutic drug product is a low pH treatment step or a treatment step with a virus removal medium (refer to, for example, Non-Patent Document 2). The treatment step with a virus removal medium such as virus removal membrane is superior because it can be applied to all the viruses whether they are enveloped or not.

In a viral clearance test in the production steps of a biological material-containing therapeutic drug product, a virus concentration is typically measured before and after an arbitrary production step and a viral clearance capacity is evaluated, for example, by calculating a log reduction value (LRV) of the virus concentration (refer to, for example, Non-Patent Document 1). For example, the viral clearance capacity in a virus removal step is evaluated by mixing a biological material-containing solution with a virus solution and measuring the respective virus concentrations of the mixture before and after the mixture is brought into contact with a virus removal medium.

With regard to the kind of a virus to be used in evaluating a viral clearance capacity, a guideline is described in q5a of ICT (International Conference on Harmonization of Technical Requirements for Human Use in Europe, the United States, and Japan) and "animal parvoviruses" are given as an example of a useful and nonspecific model virus (refer to, for example, Non-Patent Document 1). Among them, minute virus of mice (MVM) and porcine parvovirus (PPV) are frequently used in many viral clearance tests which have quoted the ICH-q5a.

For measuring the virus concentration, mainly used are an infectivity titer measurement method including an endpoint assay method and a local lesion calculation assay method and a quantitative PCR (Quantitative-Polymerase Chain Reaction: qPCR) method (refer to, for example, Non-Patent Documents 2 and 3). HERBERT LUTZ ET AL: "Qualification of a novel inline spiking method for virus filter validation",BIOTECHNOLOGY PROGRESS, AMERICAN CHEMICAL SOCIETY, HOBOKEN, USA, vol. 27, no. 1, 27 September 2010 (2010-09-27), pages 121-128, WO 2022/072899 A1 (REGENERON PHARMA [US]) 7 April 2022 (2022-04-07), BOHONAK DAVID M. ET AL: "Adapting virus filtration to enable intensified and continuous monoclonal antibody processing",BIOTECHNOLOGY PROGRESS,vol. 37, no. 2 11 December 2020 (2020-12-11), YING LI ET AL: "Novel spiking methods developed for anion exchange chromatography operating in a continuous process",BIOTECHNOLOGY AND BIOENGINEERING, JOHN WILEY, HOBOKEN, USA, vol. 117, no. 11, 30 July 2020 (2020-07-30), pages 3379-3389, and Anonymous: "Viral Safety - Practical Solutions for Risk Control",, 1 June 2018 (2018-06-01), pages 1-39, URL:https://www.pall.com/content/dam/pall/biopharm/lit-library/gated/special/19-07511-USD3364-Virus-Safety-Practical-Solutions-Risk-Control-BOOK-EN.PDF all disclose inline spiking tests.

Citation List

Patent Documents

    Patent Document 1: WO2014/080676A1
    Patent Document 2: US Patent Application Publication No. 2012/0088228
    Patent Document 3: Japanese Patent No. 4024041

Non-Patent Documents

Non-Patent Document 1: Virul Safety Evalation of Biotechnology Product Derived from Celline of Human or Animal Origin Q5A (R1)
Non-Patent Document 2: Note For Guidance On Virus Validation Studies: The Design, Contribution And Interpretation Of Studies Validating The Inactivation and Removal Of Viruses
Non-Patent Document 3: Guideline for safety guarantee of plasma fractionated product against virus (Report Number 1047 of the Pharmaceutical and Food Safety Bureau, on August 30, 1999, the notification of the director of the Pharmaceutical and Medical Safety Bureau)
Non-Patent Document 4: Raphael Wolfisberg et al., Journal of Virology (2016)
Non-Patent Document 5: Beatriz Maroto et al., JOURNAL OF VIROLOGY (2004)
Non-Patent Document 6: Ed. by Students' Association/Japanese National Institute of Health, Virus Experiments, particulars: pp. 22-23
Non-Patent Document 7: V. Hutornojs at. al (2012) Env, Exp.
Non-Patent Document 8: Anthony M. D'Abramo Jr. et al., 2005 Virology
Non-Patent Document 9: Joshua C Grieger et al., Molecular Therapy 2015
Non-Patent Document 10: Pavel Plevka et al., JOURNAL OF VIROLOGY (2011)
Non-Patent Document 11: PETER TATTERSALL et al., JOURNAL OF VIROLOGY (1976)

**Summary of Invention**

Technical Problem

The present inventors have thought that a viral clearance test method applicable to a continuous process is useful. One of the subjects of the present invention is therefore to provide a viral clearance test method applicable to a continuous process.

Solution to Problem

[1] A viral clearance test method, comprising:

supplying a protein solution to a first channel provided with, upstream and downstream thereof, a protein purification unit and a virus removal filter, respectively, and pouring the protein solution into the protein purification unit at a first constant rate;
supplying a virus solution, at a second constant rate, to a second channel connected between the protein purification unit and the virus removal filter in the first channel and mixing the purified protein solution with the virus solution in the first channel;
pouring a mixture of the protein solution and the virus solution into the virus removal filter at a third constant rate; and measuring a virus contained in a permeate of the mixture which has passed through the virus removal filter, wherein supposing that a represents the first constant rate,
b represents the second constant rate, x represents a virus concentration in the mixture, and y represents a virus concentration in the virus solution, a, b, x, and y satisfy the following formula (1): $x/y = b/(a+b)$ (1); and
wherein supposing that $C$ ($m^2$) represents a membrane area of the virus removal filter, $D_{min}$ represents a minimum value of $b/(a+b)$, $D_{max}$ represents a maximum value of $b/(a+b)$, $F_{min}$ (LMH) 2 represents a minimum flux of the permeate in the virus removal filter, and $F_{max}$ (LMH) represents a maximum flux of the permeate in the virus removal filter,
a minimum value $a_{min}$ (mL/min) of the first constant rate a is given by the following formula (2):

$$a_{min} = (1 - D_{max})\ (1000/60) \times F_{min} \times C\ (2),$$

a maximum value $a_{max}$ (mL/min) of the first constant rate a is given by the following formula (3): $a_{max} = (1-D_{min})(1000/60) \times F_{max} \times C$ (3),
a minimum value $b_{min}$ (mL/min) of the second constant rate b is given by the following formula (4): $b_{min} = D_{min}(1000/60) \times F_{min} \times C$ (4), and
a maximum value $b_{max}$ (mL/min) of the second constant rate b is given by the following formula (5): $b_{max} = D_{max}(1000/60) \times F_{max} \times C$ (5) .

[2] The method according to [1], wherein the first channel is provided with a first pump for pouring the protein solution into the protein purification unit at the first constant rate.

[3] The method according to [1] or [2], wherein the second channel is provided with a second pump for pouring the virus solution at the second constant rate.

[4] The method according to any of [1] to [3], wherein the protein solution is continuously poured into the protein purification unit.

[5] The method according to any of [1] to [4], wherein the virus solution is continuously poured into the second channel.

[6] The method according to any of [1] to [5], wherein the mixture is continuously poured into the virus removal filter.

[7] The method according to any of [1] to [6], wherein a ratio of the second constant rate to a sum of the first constant rate and the second constant rate is 0.1% or more and 20% or less.

[8] The method according to any of [1] to [7], wherein a virus infectivity titer ($Log_{1C}$ $TCID_{50}$ (unit/mL)) in the mixture is 2 or more and 10 or less.

[9] The method according to any of [1] to [8] wherein the virus removal filter has a membrane area of 0.0001 $m^2$ or more and 4 $m^2$ or less.

[10] The method according to any of [1] to [9] wherein a flux of the permeate in the virus removal filter is 0.1 LMH or more and 500 LMH or less.

[11] The method according to any of [1] to [10], further including: after pausing the supply of the protein solution into the first channel, supplying the first channel with a washing liquid and pouring the washing liquid into the protein purification unit and the virus removal filter; and measuring a virus contained in a permeate of the washing liquid which has passed through the virus removal filter.

[12] The method according to [11], wherein time until the first channel is supplied with the washing liquid after the supply of the protein solution into the first channel is paused is 0 minutes or more and 24 hours or less.

[13] The method according to any of [1] to [12], wherein the virus solution contains a protein.

[14] The method according to any of [1] to [13], wherein the virus solution contains a protein the same as the protein contained in the protein solution.

[15] The method according to [13] or [14], wherein a concentration of the protein in the virus solution is equal to a concentration of the protein in the protein solution.

[16] The method according to any of [1] to [15], further including comparing an amount of the virus contained in the mixture before passing through the virus removal filter with the amount of the virus contained in the permeate of the mixture which has passed through the virus removal filter.

[17] The method according to any of [1] to [16], wherein the protein purification unit has a virus removal capacity.

[18] The method according to [17], wherein a log reduction value (LRV) in the protein purification unit 11 is 0 or more and 7 or less.

Advantageous Effects of Invention

The present invention makes it possible to provide a viral clearance test method applicable to a continuous process.

**Brief Description of Drawings**

Fig. 1 is a schematic view of the protein purification system of an embodiment.
Fig. 2 is a table showing the purification conditions of Examples 1 to 6.
Fig. 3 is a table showing the purification results of Examples 1 to 6.
Fig. 4 is a schematic view of the protein purification system of Comparative Example 1.
Fig. 5 is a table showing the purification conditions of Comparative Examples 1 to 3.
Fig. 6 is a table showing the purification results of Comparative Examples 1 to 3.
Fig. 7 is a schematic view of the protein purification system of Comparative Example 4.
Fig. 8 is a table showing the purification conditions of Comparative Examples 4 to 9.
Fig. 9 is a table showing the purification results of Comparative Examples 4 to 9.

Description of Embodiments

A mode (which may hereinafter be called "embodiment") for carrying out the present invention will hereinafter be described. The embodiments shown below are only examples of the method for embodying the technical concept of the present invention and the present invention is not limited by these examples.

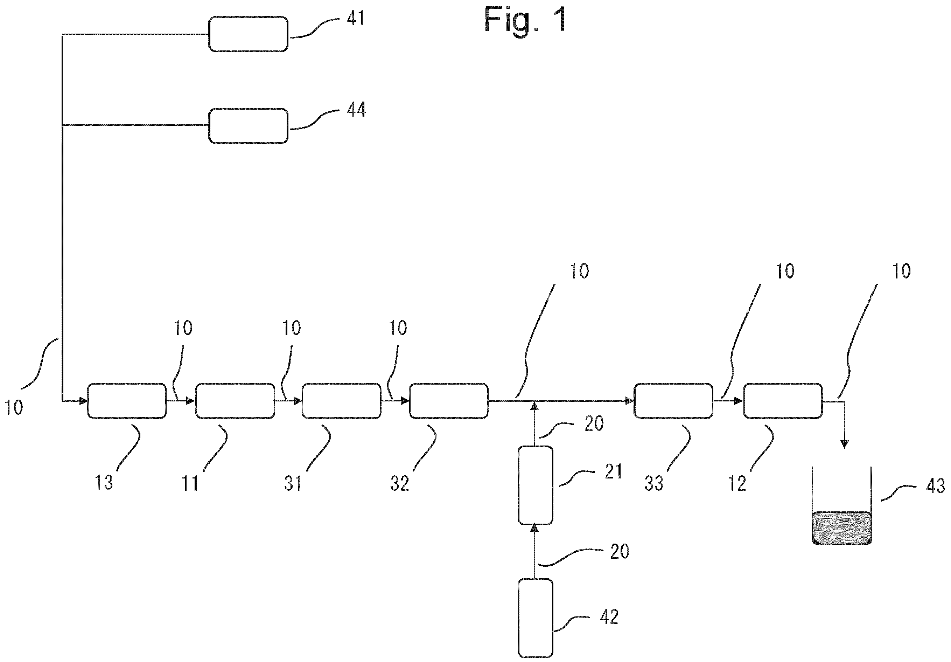

The protein purification system according to the present embodiment has, as shown in Fig. 1, a first channel 10 through which a protein solution flows, a protein purification unit 11 provided upstream of the first channel 10, a virus removal filter 12 provided downstream of the first channel 10, and a second channel 20 connected between the protein purification unit 11 and the virus removal filter 12 in the first channel 10. The first channel 10 may be provided with a first pump 13 for pouring the protein solution into the protein purification unit 11 at a first constant rate. The second channel 20 may be provided with a second pump 21 for pouring a virus solution at a second constant rate.

The method of the viral clearance test method according to the present embodiment is performed using, for example, the protein purification system shown in Fig. 1. The method of the viral clearance test method according to the present embodiment includes (a) supplying a protein solution to a first channel 10 provided with, upstream and downstream thereof, a protein purification unit 11 and a virus removal filter 12, respectively, and pouring the protein solution into the protein purification unit 11 at a first constant rate; (b) supplying a virus solution, at a second constant rate, to a second channel 20 connected between the protein purification unit 11 and the virus removal filter 12 in the first channel 10 and mixing the purified protein solution with the virus solution in the first channel 10; (c) pouring a mixture of the protein solution and the virus solution into the virus removal filter 12 at a third constant rate; and (d) measuring a virus contained in a permeate of the mixture which has passed through the virus removal filter 12.

The protein solution to be poured into the first channel 10 contains a protein. The protein solution preferably does not contain a virus. The protein solution is, for example, poured into the first channel 10 from a protein solution tank 41.

The first pump 13 is provided, for example, upstream of the protein purification unit 11 in the first channel 10 but is not limited thereto. As the first pump 13, a volume pump can be used, but it is not limited thereto. Examples of the volume pump include, but not limited to, a peristaltic pump. The first pump 13 continuously pours the protein solution into the protein purification unit 11 at a first constant rate.

Examples of the protein include antibodies. An antibody is, as generally defined in biochemistry, a glycoprotein molecule (also called "gamma globulin" or "immunoglobulin") produced by B-lymphocytes as an infection protective mechanism of vertebrate animals. For example, an antibody is used as a pharmaceutical product for humans and may have substantially the same structure as that of an antibody in the human body to which it is administered.

The antibody may be a human antibody or an antibody derived from a nonhuman mammal such as bovine and mouse. Alternatively, the antibody may be a chimeric antibody with human IgG or a humanized antibody. The chimeric antibody with human IgG is an antibody in which variable regions are derived from a nonhuman organism such as mouse and the other constant regions are replaced by human-derived immunoglobulin. The humanized antibody is an antibody having a variable region in which a complementarity-determining region (CDR) is derived from a nonhuman organism and the other framework region (FR) is a human-derived one. The humanized antibody has further reduced immunogenicity than the chimeric antibody.

The class (isotype) and subclass of the antibody are not limited. For example, the antibody is classified according to a difference in the structure of a constant region into five classes such as IgG, IgA, IgM, IgD, and IgE. However, the antibody may belong to any of these five classes. In the human antibody, IgG has four subclasses IgG1 to IgG4 and IgA has two subclasses IgA1 and IgA2. The antibody may belong to any of these subclasses. An antibody-related protein such as Fc fusion protein obtained by binding a protein to a Fc region may also be included in the antibody.

The antibody can be classified according to its origin. However, the antibody may be any of a naturally occurring human antibody, a recombinant human antibody produced by genetic recombination technique, a monoclonal antibody, and a polyclonal antibody. From the standpoint of demand and importance as an antibody drug, human IgG is preferred as an antibody but is not limited thereto.

The protein purification unit 11 removes an impurity contained in the protein and purifies the protein contained in a protein solution. The protein purification unit 11 is equipped with, for example, a chromatography column.

The chromatography column may be, for example, a cation exchange chromatography column. In the cation exchange chromatography column, an aggregate of a protein such as an oligomer in the antibody is adsorbed, as an impurity, to a cation exchange carrier and a protein such as a monomer in the antibody passes through the cation exchange chromatography column.

The cation exchange carrier has a cation exchange group. The cation exchange group may be either a strong cation exchange group or a weak cation exchange group, or it may be both.

The strong cation exchange group generally shows a constant charge amount because it is charged in a pH region of an antibody solution. Accordingly, when the cation exchange carrier has a strong cation exchange group, at least a predetermined charge amount is constantly ensured. When the cation exchange carrier has a strong cation exchange group, a change in charge amount relative to pH is suppressed and improvement in reproducibility of purification properties can be achieved. Examples of the strong cation exchange group include a sulfonic acid group.

The weak cation exchange group can change a charge amount by the pH of a mobile phase. By changing the pH of a mobile phase, therefore, the charge density of the cation exchange carrier can be controlled. This means that by adjusting the pH according to the properties of an impurity to be removed, any intended impurity can be removed. Examples of the weak cation exchange group include a carboxyl group, a phosphonic acid group, and a phosphoric acid group.

Examples of the form of the cation exchange carrier include, but not limited to, membrane, beads, and monolith.

Examples of the cation exchange carrier in the form of membrane include, but not limited to, Mustang (trade name) S (Pall Corporation), Sartobind (trademark) S (Sartorius Stedim Biotech), and Natrix HD-Sb and Natrix HD-C (each, Natrix Separations).

Examples of the cation exchange carrier in the form of beads include, but not limited to, SP Sepharose (trade name) Fast Flow, High Performance, and XL; Capto (trade name) S (GE Healthcare), Fractogel (trademark) COO-, SO$_3$-, and SE Highcap; Eshumuno (trademark) S and CPX (Merck Millipore Corporation); POROS (trademark) XS and HS (ThermoFisher), Nuvia (trade name) S and HR-S; UNOsphere (trade name) S and Rapid S; Macro-Prep (trademark) High S, CM, and 25 S (Bio-Rad); and Cellufine (trademark) Max CM and Max S (JNC); and Cellufine (trademark) DexS-HbP (JNC).

Examples of the cation exchange carrier in the form of monolith include, but not limited to, CIM (trademark) SO3 (BIA Separations).

The chromatography column may be, for example, an anion exchange chromatography column. In the anion exchange chromatography column, an impurity having a low isoelectric point such as host cell-derived protein (HCP), nucleic acid, or virus adsorbs to an anion exchange carrier and a protein such as a monomer in an antibody passes through the anion exchange chromatography column.

The anion exchange carrier has an anion exchange group. The anion exchange group may be either a strong anion exchange group or a weak anion exchange group, or it may be both.

Examples of the strong anion exchange group include quaternary ammonium having a trimethylamino group, a triethylamino group, or the like.

Examples of the weak anion exchange group include, but not limited to, a tertiary amine. A tertiary amine having two or more alkyl groups having two or more carbon atoms may have adequate hydrophobicity. Examples of the tertiary amine include a diethylamino group, a dipropylamino group, a diisopropylamino group, and a dibutylamino group.

Examples of the shape of the cation exchange carrier include, but not limited to, membrane, beads, and monolith.

Examples of the anion exchange carrier in the form of membrane include, but not limited to, Chromasorb (trade name) (Merck Millipore Corporation), Mustang (trademark) Q (Pall Corporation), Sarotibind (trademark) Q, STIC (trademark) PA (Sartorius Stedim Biotech), NatriFlo (trademark) HD-Q (Natrix Separations), and QyuSpeed (trade name) D (Asahi Kasei Medical).

Examples of the anion exchange carrier in the form of beads include, but not limited to, Q Sepharose (trade name) Fast Flow, High Performance, and XL; QAE Sephadex (trade name) (GE Healthcare); Fractogel (trade mark) TMAE, TMAE Highcap, DMAE, and DEAE; Eshmuno (trade mark) Q (Merck Millipore Corporation); POROS (trademark) XQ, HQ, D, and PI (ThermoFisher); DEAE-Cellulose (Sigma-Aldrich); Nuvia (trade name) Q; UNOsphere (trade name) Q; Macro-Prep (trademark) High Q, DEAE, and 25 Q (Bio-Rad); CaptoQ (GE Health Care Japan); and Cellufine (trademark) Max DEAE and Max Q (JNC).

Examples of the anion exchange carrier in the form of monolith include, but not limited to, CIM (trademark) QA, DEAE, and EDA (BIA Separations).

The chromatography column may be, for example, a mixed mode chromatography column. In the mixed mode chromatography column, reverse phase chromatography and ion exchange chromatography are used in combination to purify the protein solution. Examples of a carrier used in the mixed mode chromatography include Cellufine MAX IB (JNC).

The protein purification unit 11 may have a virus removing capacity or may not have a virus removing capacity. A log reduction value (LRV) in the protein purification unit 11 may be, for example, 0 or more and 7 or less. The lower limit of LRV in the protein purification unit 11 may be 0 or more, 1 or more, 2 or more, 3 or more, 4 or more, or 5 or more. The upper limit of LRV in the protein purification unit 11 may be 7 or less or 6 or less.

The protein purification unit 11 in the first channel 10 may be provided with, downstream thereof, a protein concentration measuring instrument 31 for measuring the protein concentration of the protein solution which has passed through the protein purification unit 11. The protein concentration measuring instrument 31 measures the protein concentration of the protein solution which has passed the protein purification unit 11, for example, by the ultraviolet absorption method.

The protein purification unit 11 in the first channel 10 may be provided with, downstream of the unit, a conductivity measuring instrument 32 for measuring the conductivity of the protein solution which has passed through the protein purification unit 11. The conductivity measuring instrument 32 measures the conductivity of the protein solution which has passed through the protein purification unit 11, for example, by the AC bipolar method or electromagnetic induction method.

The protein purification unit 11 in the first channel 10 may also be provided with, downstream of the unit, a pH meter for measuring the pH of the protein solution which has passed through the protein purification unit 11, a thermometer for measuring the temperature, and a pressure gauge for measuring the pressure.

The virus solution to be poured in the second channel 20 contains a virus. The virus solution is, for example, poured into the second channel 20 from a virus solution tank 42.

A volume pump is usable as the second pump 21 but the second pump is not limited to it. Examples of the volume pump include, but not limited to a peristaltic pump. The second pump 21 continuously pours the virus solution into the second channel 20 at a second constant rate. The second channel 20 is connected to the first channel 10 so that the protein solution and the virus solution are mixed in the first channel 10 downstream from a connecting point of the second channel 20 and the first channel 10. An in-line mixer 33 may be provided in the first channel 10 downstream from the connecting point of the second channel 20 and the first channel 10. The in-line mixer 33 accelerates the mixing of the protein solution and the virus solution.

The virus may be an infectious virus. The virus may be a naturally-occurring virus. The naturally-occurring virus includes a virus obtained by culturing, in a medium, a host cell infected with a virus and a virus obtained by transfecting a virus nucleic acid into a cell and culturing the resulting cell.

Examples of the virus include, but not limited to, minute virus of mice (MVM), porcine parvovirus (PPV), reovirus type 3, acute poliomyelitis virus (PolioVirus), porcine herpes virus (Pseudorabies Virus), herpes simplex virus type 1 (Human Herpes Virus 1), xenotropic murine leukemia virus (X-MuLV), and bovine viral diarrhea virus.

For example, the virus solution to be supplied to the second channel 20 contains a protein the same as that contained in the protein solution to be supplied to the first channel 10. For example, the concentration of the protein in the virus solution to be supplied in the second channel 20 is equal to that of the protein in the protein solution to be supplied in the first channel 10. The concentration of the protein in the mixture of the protein solution and the virus solution therefore becomes equal to the concentration of the protein in the protein solution supplied in the first channel 10.

The infectivity titer ($Log_{10} TCID_{50}$ (unit/mL)) of the virus in the mixture of the protein solution and the virus solution to be poured into the virus removal filter 12 is, for example, 2 or more, 3 or more, or 4 or more. The infectivity titer ($Log_{10} TCID_{50}$ (unit/mL)) of the virus in the mixture is, for example, 10 or less, 9 or less, 8 or less, or 7 or less.

Supposing that **a** represents the first constant rate, **b** represents the second constant rate, **x** represents a virus concentration of the mixture, and **y** represents a virus concentration in the virus solution, the first constant rate, the second constant rate, the supply amount of the protein solution, the supply amount of the virus solution, and the virus concentration in the virus solution may be adjusted so that **a, b, x,** and **y** satisfy the following formula (6):

$$x/y = b/(a+b) \quad (6)$$

A ratio of the second constant rate to the sum of the first constant rate and the second constant rate is, for example, 0.1% or more, 0.5% or more, 1.0% or more, 1.5% or more, 2.0% or more, or 3.0% or more. A ratio of the second constant rate to the sum of the first constant rate and the second constant rate is, for example, 20% or less, 15% or less, 10% or less, 9% or less, 8% or less, or 7% or less. By adjusting the ratio of the second constant rate to the sum of the first constant rate and the second constant rate to fall within the aforesaid range, the flow of the protein solution is less likely to be influenced largely by the flow of the virus solution.

The mixture of the protein solution and the virus solution continuously flows into the virus removal filter 12 at a third constant rate.

The membrane area of the virus removal filter 12 is, for example, 0.0001 $m^2$ or more, 0.0002 $m^2$ or more, 0.0003 $m^2$ or more, 0.0006 $m^2$ or more, 0.0009 $m^2$ or more, or 0.0015 $m^2$ or more. The membrane area of the virus removal filter 12 is, for example, 4 $m^2$ or less, 3 $m^2$ or less, 2 $m^2$ or less, or 1 $m^2$ or less.

The virus removal filter 12 may be in the form of a hollow yarn or a flat membrane.

Examples of the virus removal filter in the form of a hollow yarn include Planova 15N, 20N, and 35N, and BioEx (each, product of Asahi Kasei Medical).

Examples of the virus removal filter in the flat membrane form include Viresolve Pro (EMD Millipore Corporation); Ultipor VF Grade DV20 and DV50 and Pegasus (trade name) SV4 and Grade LV6 (Pall Corporation); Virosart CPV, HC, and HF (Sartorius Stedim Biotech), and NFP (Merck Millipore Corporation).

The flux of the permeate in the virus removal filter 12 is, for example, 0.1 LMH or more, 1.0 LMH or more, 2.0 LMH or more, 4.0 LMH or more, or 10.0 MHL or more. The flux of the permeate in the virus removal filter 12 is, for example, 500 LMH or less, 400 LMH or less, 300 LMH or less, 200 LMH or less, or 100 LMH or less. The flux of the permeate in the virus removal filter 12 is adjusted by the first pump 13 and the second pump 21.

Supposing that C ($m^2$) represents a membrane area of the virus removal filter 12, a represents the first constant rate, b represents the second constant rate, $D_{min}$ represents a minimum value of b/(a+b), $D_{max}$ represents a maximum value of b/(a+b), $F_{min}$ (LMH) represents a minimum flux of the permeate in the virus removal filter, and $F_{max}$ (LMH) represents a maximum flux of the permeate in the virus removal filter, a minimum value $a_{min}$ (mL/min) of the first constant rate a is given, for example, by the following formula (7):

$$a_{min} = (1 - D_{max})(1000/60) \times F_{min} \times C \quad (7),$$

A maximum value $a_{max}$ (mL/min) of the first constant rate a is given, for example, by the following formula (8):

$$a_{max} = (1-D_{min}) \ (1000/60) \ \times \ F_{max} \ \times \ C \quad (8),$$

A minimum value $b_{min}$ (mL/min) of the second constant rate b is given, for example, by the following formula (9):

$$b_{min} = D_{min}(1000/60) \ \times \ F_{min} \ \times \ C \quad (9).$$

A maximum value $b_{max}$ (mL/min) of the second constant rate b is given, for example, by the following formula (10):

$$b_{max} = D_{max}(1000/60) \ \times \ F_{max} \ \times \ C \quad (10).$$

The protein solution supplied in the first channel 10 is continuously poured into the first channel 10 provided with the protein purification unit 11 and the virus removal filter 12. The virus solution supplied in the second channel 20 is continuously poured into the second channel 20 and the first channel 10 provided with the virus removal filter 12. The term "continuously poured" means that the solution is poured without pooling the solution in the middle of the channel.

The permeate of the mixture which has passed the virus removal filter 12 is collected, for example, in a permeate collecting container 43. Examples of a method of measuring the virus contained in the permeate of the mixture which has passed through the virus removal filter 12 include, but not limited to, an infectivity titer measurement method and a quantitative PCR method.

The infectivity titer in the infectivity titer measurement method is a unit expressing the concentration of a virus having infectivity. The infectivity titer measurement method includes an end-point assay which determines the minimum infection unit and a count assay of local lesions formed by a virus. As the end-point assay, commonly used is a 50% infection endpoint ($TCID_{50}$: Tissue culture infectious dose 50) method for determining a dilution ratio at which 50% of the cells are infection positive. It is determined by carrying out stepwise dilution of a virus, inoculating it on at least a predetermined number of culture cells, culturing them for a predetermined term, and judging infection positive/negative. As the local lesion count assay, commonly used is a plaque assay, which determines plaques formed when inoculating a virus to cells cultured in sheet form, overlaying an agar-containing medium to cover the cells, and measuring the number of plaques formed corresponding to the number of inoculated viruses. The infectivity titer is expressed by $TCID_{50}$ when the $TCID_{50}$ method is used and by pfu when the plaque assay is used. The pfu is an abbreviation of a plaque forming unit. The infectivity titer per mL is expressed by a unit $TCID_{50}$ /mL or pfu/mL.

By the quantitative PCR, a nucleic acid enclosed in a virus is quantitatively determined. Typically, one virus particle encloses one nucleic acid molecule in a capsid so that the number of nucleic acid molecules becomes equal to the number of virus particles.

Based on the amount of the virus contained in the mixture before passing through the virus removal filter 12 and the amount of the virus contained in the mixture which has passed through the virus removal filter 12, the viral clearance capacity of the virus removal filter 12 is evaluated. The amount of the virus may be expressed by an infectivity titer or the number of particles. The viral clearance capacity of the virus removal filter 12 is evaluated, for example, by a log reduction value (LRV) given by the following formula (11):

$$LRV = Log_{10}T_1 - Log_{10}T_2 \quad (11)$$

In the above formula, $T_1$ represents an amount of a virus contained in the mixture before passing through the virus removal filter 12 and $T_2$ represents an amount of a virus contained in the mixture which has passed through the virus removal filter 12.

When the LRV is larger, the virus removal filter 12 can be evaluated as having a higher viral clearance capacity.

After the supply of the first channel 10 with the protein solution is paused, the first channel 10 may be supplied with a washing liquid. The washing liquid is poured, for example, from a washing liquid tank 44 to the first channel 10. The washing liquid is a solvent containing neither a protein nor a virus. The washing liquid is poured into the protein purification unit 11 and the virus removal filter 12 and the amount of the virus contained in the permeate of the washing liquid which has passed through the virus removal filter 12. The virus removal filter 12 can be evaluated as having a high virus retaining capacity when the amount of the virus contained in the permeate of the washing liquid is small.

The time until the washing liquid is supplied to the first channel 10 after the supply of the first channel 10 with the protein solution is paused is, for example, 0 minutes or more, 5 minutes or more, 10 minutes or more, or 30 minutes or more. The time (process pause) until the washing liquid is supplied to the first channel 10 after the supply of the first channel 10 with the protein solution is paused is, for example, 24 hours or less, 20 hours or less, 10 hours or less, 5 hours or less, or 1 hour or less.

The method of the viral clearance test method according to the present embodiment makes it possible to carry out a viral clearance test with high reproducibility because virus loading to the virus removal filter 12 can be conducted under uniform conditions by supplying the second channel 20 with the virus solution at a constant rate. When the second channel 20 is supplied with the virus solution not at a constant rate, the reproducibility of the viral clearance test may lower. In addition, when the second channel 20 is supplied with the virus solution not at a constant rate, a change in virus spike amount may make it impossible to calculate the log reduction value (LRV).

(Example 1)

A system similar to the protein purification system as shown in Fig. 1 was manufactured. As the protein purification unit 11, a 0.5 mL column packed with a mixed mode chromatography carrier (Cellufine MAX IB, JNC) was used. The Cellufine MAX IB has a ligand obtained by partially modifying a polyamine with a butyl group. As the virus removal filter 12, a Planova BioEx (Asahi Kasei Medical) having a membrane area of 0.0003 $m^2$ was used.

A protein solution containing 5 mg/mL IgG was prepared using a solvent of pH 6.5 containing 20 mmol/L tris-acetic acid and 100 mmol/L NaCl. A virus solution containing 10% MVM was prepared by adding MVM to the protein solution.

With the first pump 13, 27 mL of the protein solution was poured into the first channel 10 at a first constant rate of 0.225 mL/min. With the second pump 21, 3 mL of the virus solution was poured into the second channel 20 at a second constant rate of 0.025 mL/min. The percentage of the second constant rate in the sum of the first constant rate and the second constant rate was 10%. As a result, a mixture having a virus infectivity titer ($Log_{10}$ $TCID_{50}$ (unit/mL)) of 6.884 was poured into the virus removal filter 12 and the flux of the permeate was 50 LHM. The permeate of the mixture was collected.

After the 30 mL solution was poured into the virus removal filter 12, the first pump 13 and the second pump 21 were paused and the system was allowed to stand for 35 minutes. Then, a solvent of pH 6.5 containing 20 mmol/L tris-acetic acid and 100 mmol/L NaCl used as a washing liquid was poured into the first channel 10 at a constant rate of 0.25 mL/min with the first pump 13. A permeate of the washing liquid which had passed through the protein purification unit 11 and the virus removal filter 12 was collected.

A virus infectivity titer in the collected permeate of the mixture was measured. A log reduction value (LRV) in the virus removal filter 12 as calculated from the virus infectivity titer in the permeate of the mixture and the virus infectivity titer in the mixture before passing through the virus removal filter 12 was 5.56 or more.

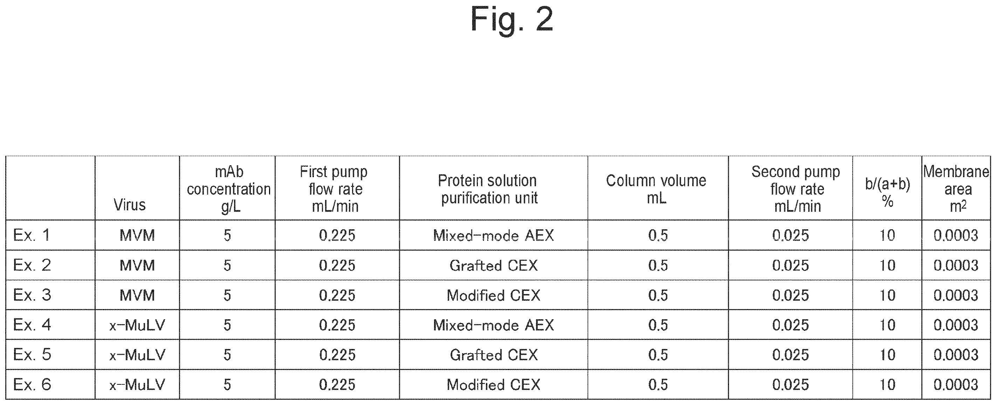

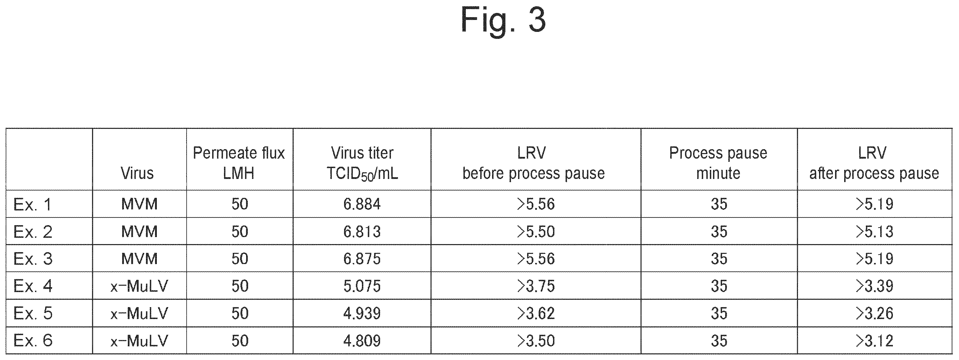

A virus infectivity titer in the collected permeate of the washing liquid was measured. A log reduction value (LRV) in the virus removal filter 12 as calculated from the virus infectivity titer in the permeate of the mixture, the virus infectivity titer in the permeate of the washing liquid, and the virus infectivity titer in the mixture before passing through the virus removal filter 12 was 5.19 or more. The purification conditions and the purification results in Example 1 are shown in Fig. 2 and Fig. 3, respectively.

(Example 2)

In a manner similar to that of Example 1 except for the use of a 0.5 mL column packed with a strong cation exchange chromatography carrier (Cellufine MAX GS, JNC) as the protein purification unit 11, the protein solution and the virus solution were poured into the protein purification system. The resulting mixture had a virus infectivity titer ($Log_{10}$ $TCID_{50}$ (unit/mL)) of 6.813.

A log reduction value (LRV) in the virus removal filter 12 as calculated from a virus infectivity titer in a permeate of the mixture and the virus infectivity titer in the mixture before passing through the virus removal filter 12 was 5.50 or more.

A log reduction value (LRV) in the virus removal filter 12 as calculated from the virus infectivity titer in the permeate of the mixture, a virus infectivity titer in a permeate of the washing liquid, and the virus infectivity titer in the mixture before passing through the virus removal filter 12 was 5.13 or more. The purification conditions and the purification results in Example 2 are shown in Fig. 2 and Fig. 3, respectively.

(Example 3)

In a manner similar to that of Example 1 except for the use of a 0.5 mL column packed with a strong cation exchange chromatography carrier (Cellufine DexS-HbP, JNC) as the protein purification unit 11, the protein solution and the virus solution were poured into the protein purification system. The resulting mixture had a virus infectivity titer ($Log_{10}$ $TCID_{50}$ (unit/mL)) of 6.875.

A log reduction value (LRV) in the virus removal filter 12 as calculated from a virus infectivity titer in a permeate of the mixture and the virus infectivity titer in the mixture before passing through the virus removal filter 12 was 5.56 or more.

A log reduction value (LRV) in the virus removal filter 12 as calculated from the virus infectivity titer in the permeate of the mixture, a virus infectivity titer in a permeate of the washing liquid, and the virus infectivity titer in the mixture before passing through the virus removal filter 12 was 5.19 or more. The purification conditions and the purification results in Example 3 are shown in Fig. 2 and Fig. 3, respectively.

(Example 4)

In a manner similar to that of Example 1 except for the use of a virus solution containing 10% x-MuLV, the protein solution and the virus solution were poured into the protein purification system. The resulting mixture had a virus infectivity titer ($Log_{10}$ $TCID_{50}$ (unit/mL)) of 5.075.

A log reduction value (LRV) in the virus removal filter 12 as calculated from a virus infectivity titer in a permeate of the mixture and the virus infectivity titer in the mixture before passing through the virus removal filter 12 was 3.75 or more.

A log reduction value (LRV) in the virus removal filter 12 as calculated from the virus infectivity titer in the permeate of the mixture, a virus infectivity titer in a permeate of the washing liquid, and the virus infectivity titer in the mixture before passing through the virus removal filter 12 was 3.39 or more. The purification conditions and the purification results in Example 4 are shown in Fig. 2 and Fig. 3, respectively.

(Example 5)

In a manner similar to that of Example 2 except for the use of a virus solution containing 10% x-MuLV, the protein solution and the virus solution were poured into the protein purification system. The resulting mixture had a virus infectivity titer ($Log_{10}$ $TCID_{50}$ (unit/mL)) of 4.939.

A log reduction value (LRV) in the virus removal filter 12 as calculated from a virus infectivity titer in a permeate of the mixture and the virus infectivity titer in the mixture before passing through the virus removal filter 12 was 3.62 or more.

A log reduction value (LRV) in the virus removal filter 12 as calculated from the virus infectivity titer in the permeate of the mixture, a virus infectivity titer in a permeate of the washing liquid, and the virus infectivity titer in the mixture before passing through the virus removal filter 12 was 3.26 or more. The purification conditions and the purification results in Example 5 are shown in Fig. 2 and Fig. 3, respectively.

(Example 6)

In a manner similar to that of Example 3 except for the use of a virus solution containing 10% x-MuLV, the protein solution and the virus solution were poured into the protein purification system. The resulting mixture had a virus infectivity titer ($Log_{10}$ $TCID_{50}$ (unit/mL)) of 4.809.

A log reduction value (LRV) in the virus removal filter 12 as calculated from a virus infectivity titer in a permeate of the mixture and the virus infectivity titer in the mixture before passing through the virus removal filter 12 was 3.50 or more.

A log reduction value (LRV) in the virus removal filter 12 as calculated from the virus infectivity titer in the permeate of the mixture, a virus infectivity titer in a permeate of the washing liquid, and the virus infectivity titer in the mixture before passing through the virus removal filter 12 was 3.12 or more. The purification conditions and the purification results in Example 6 are shown in Fig. 2 and Fig. 3, respectively.

(Comparative Example 1)

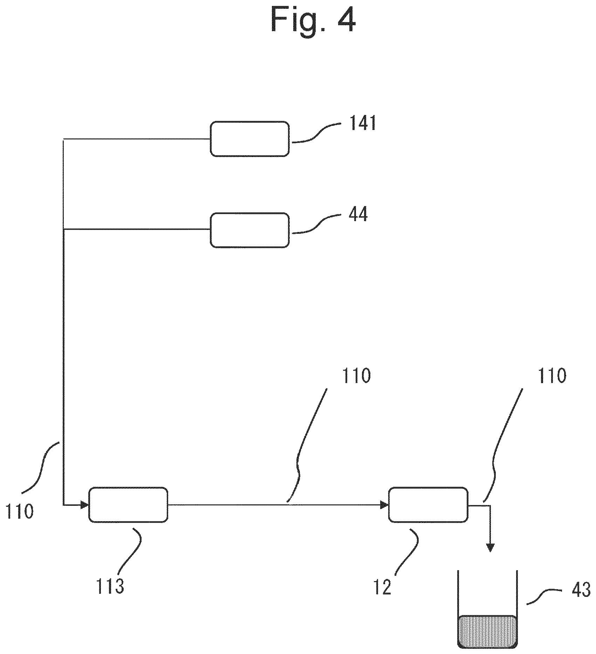

A protein purification system of Comparative Example 1 as shown in Fig. 4 was manufactured. The protein purification system of Comparative Example 1 had a channel 110, a pump 113 provided in the channel 110, and a virus removal filter 12 provided in the channel 110. As the virus removal filter 12, Planova BioEX (Asahi Kasei Medical) having a membrane area of 0.0003 $m^2$ was used. The protein purification system of Comparative Example 1 did not have a protein purification unit. In addition, the protein purification system of Comparative Example 1 did not have a second channel and a second pump.

A virus solution containing 1% MVM was prepared using materials similar to those of Example 1. With the pump 113, 30 mL of the virus solution was poured into the channel 110 at a constant rate of 0.025 mL/min, by which the virus solution having a virus infectivity titer ($Log_{10}$ $TCID_{50}$ (unit/mL)) of 6.13 flowed through the virus removal filter 12 and the flux of the permeate was 5 LHM. The permeate of the virus solution was collected.

After the 30 mL solution flowed through the virus removal filter 12, the pump 113 was paused and the system was allowed to stand for 35 minutes. Then, a washing liquid similar to that of Example 1 was poured into the channel 110 at a constant rate of 0.25 mL/min with the pump 113. A permeate of the washing liquid which had passed the virus removal filter 12 was collected.

A virus infectivity titer in the collected permeate of the virus solution was measured. A log reduction value (LRV) in the virus removal filter 12 as calculated from the virus infectivity titer in the permeate of the virus solution and the virus infectivity titer in the virus solution before passing through the virus removal filter 12 was 5.27 or more.

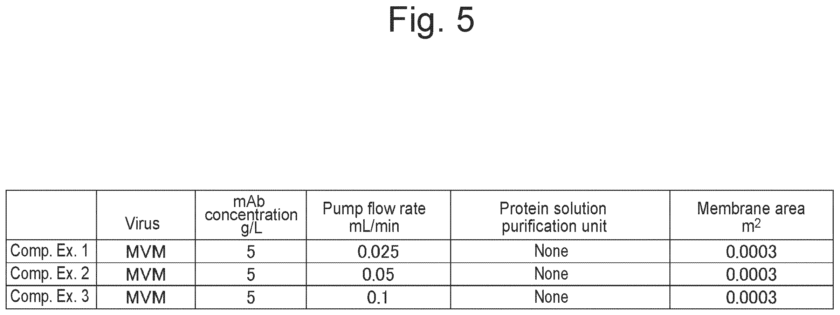

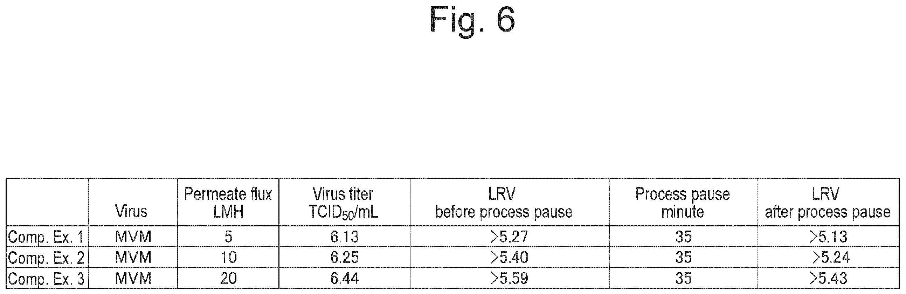

A virus infectivity titer in the collected permeate of the washing liquid was measured. A log reduction value (LRV) in the virus removal filter 12 as calculated from the virus infectivity titer in the permeate of the virus solution, the virus infectivity titer in the permeate of the washing liquid, and the virus infectivity titer in the virus solution before passing through the virus removal filter 12 was 5.13 or more. The purification conditions and the purification results in Comparative Example 1 are shown in Fig. 5 and Fig. 6, respectively.

The LRV obtained in Comparative Example 1 is approximate to the LRV obtained in Examples 1 to 3. This has revealed that in Examples 1 to 3, even when the virus removal filter 12 had, upstream thereof, the protein purification unit 11, the virus removal capacity of the virus removal filter 12 alone was tested.

(Comparative Example 2)

In a manner similar to that of Comparative Example 1 except that the virus solution was poured into the channel 110 at a constant rate of 0.05 mL/min, the virus solution was poured into the protein purification system of Comparative Example 1. The virus solution thus poured had a virus infectivity titer ($Log_{10}$ $TCID_{50}$ (unit/mL)) of 6.25 and the flux of the permeate was 10 LHM.

A virus infectivity titer in the collected permeate of the virus solution was measured. A log reduction value (LRV) in the virus removal filter 12 as calculated from the virus infectivity titer in the permeate of the virus solution and the virus infectivity titer in the virus solution before passing through the virus removal filter 12 was 5.40 or more.

A virus infectivity titer in the collected permeate of the washing liquid was measured. A log reduction value (LRV) in the virus removal filter 12 as calculated from the virus infectivity titer in the permeate of the virus solution, the virus infectivity titer in the permeate of the washing liquid, and the virus infectivity titer in the virus solution before passing through the virus removal filter 12 was 5.24 or more. The purification conditions and the purification results in Comparative Example 2 are shown in Fig. 5 and Fig. 6, respectively.

(Comparative Example 3)

In a manner similar to that of Comparative Example 1 except that the virus solution was poured into the channel 110 at a constant rate of 0.1 mL/min, the virus solution was poured into the protein purification system of Comparative Example 1. The virus solution thus poured had a virus infectivity titer ($Log_{10}$ $TCID_{50}$ (unit/mL)) of 6.44 and the flux of the permeate was 20 LHM.

A virus infectivity titer in the collected permeate of the virus solution was measured. A log reduction value (LRV) in the virus removal filter 12 as calculated from the virus infectivity titer in the permeate of the virus solution and the virus infectivity titer in the virus solution before passing through the virus removal filter 12 was 5.59 or more.

A virus infectivity titer in the collected permeate of the washing liquid was measured. A log reduction value (LRV) in the virus removal filter 12 as calculated from the virus infectivity titer in the permeate of the virus solution, the virus infectivity titer in the permeate of the washing liquid, and the virus infectivity titer in the virus solution before passing through the virus removal filter 12 was 5.43 or more. The purification conditions and the purification results in Comparative Example 3 are shown in Fig. 5 and Fig. 6, respectively.

(Comparative Example 4)

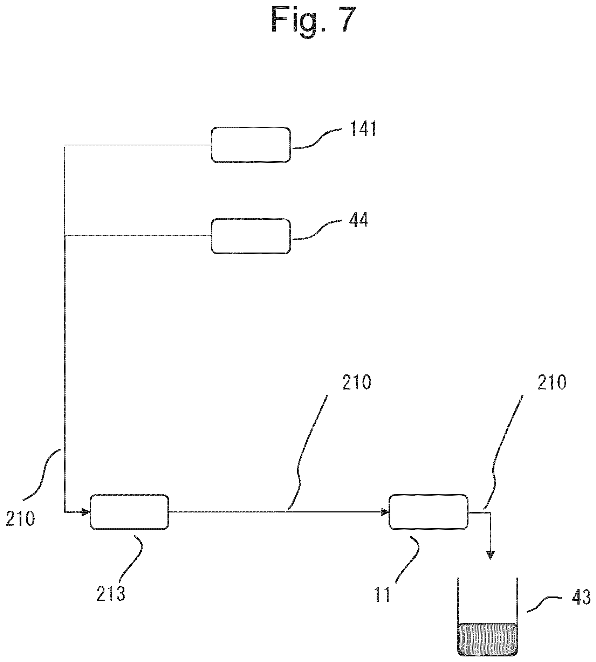

A protein purification system of Comparative Example 4 as shown in Fig. 7 was manufactured. The protein purification system of Comparative Example 4 had a channel 210, a pump 213 provided in the channel 210, and a protein purification unit 11 provided in the channel 210. As the protein purification unit 11, a 0.5 mL column packed with a mixed mode chromatography carrier (Cellufine MAX IB, JNC) was used. The protein purification system of Comparative Example 4 did not have a virus removal filter. In addition, the protein purification system of Comparative Example 4 did not have a second channel and a second pump.

A virus solution containing 5% of MVM was prepared from materials similar to those used in Example 1. The virus solution (30 mL) was poured into the channel 110 at a constant rate of 0.25 mL/min with the pump 213. As a result, the virus solution having a virus infectivity titer ($Log_{10}$ $TCID_{50}$ (unit/mL)) of 7.741 flowed through the protein purification unit 11. The permeate of the virus solution was collected.

After 30 mL of the solution flowed through the protein purification unit 11, the pump 213 was paused and the system was allowed to stand for 0.05 minutes. Then, a washing liquid the same as that of Example 1 was poured into the channel 210 at a constant rate of 0.25 mL/min with the pump 213. The permeate of the washing liquid which had passed through the protein purification unit 11 was collected.

A virus infectivity titer in the collected permeate of the virus solution was measured. A log reduction value (LRV) in the protein purification unit 11 as calculated from the virus infectivity titer in the permeate of the virus solution and the virus infectivity titer in the virus solution before passing through the protein purification unit 11 was 3.94.

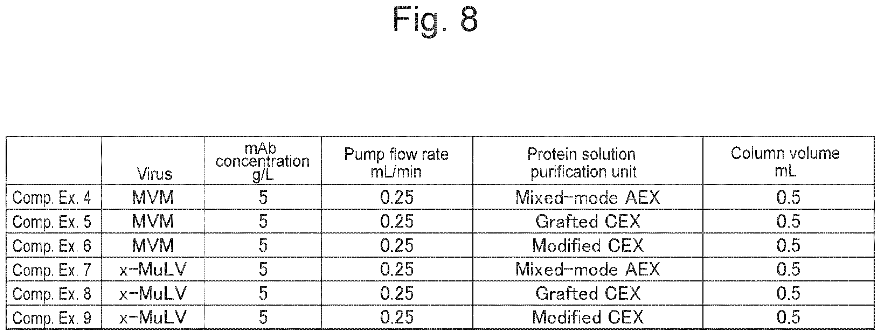

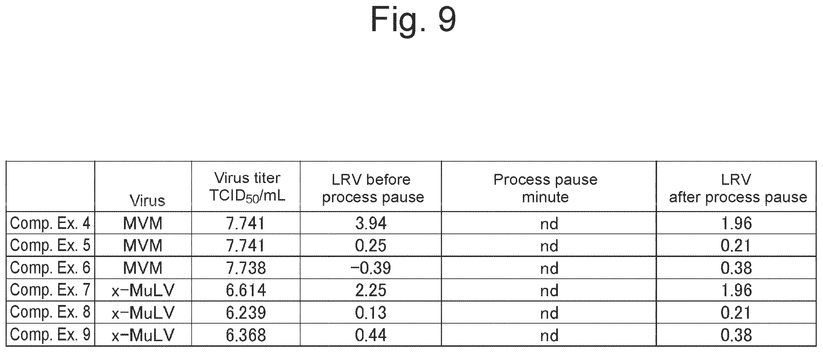

A virus infectivity titer in the collected permeate of the washing liquid was measured. A log reduction value (LRV) in the protein purification unit 11 as calculated from the virus infectivity titer in the permeate of the virus solution, the virus infectivity titer in the permeate of the washing liquid, and the virus infectivity titer in the virus solution before passing through the protein purification unit 11 was 1.96. The purification conditions and the purification results in Comparative Example 4 are shown in Fig. 8 and Fig. 9, respectively.

The above results show that the virus is removed at the protein purification unit 11. The results therefore show that when the virus solution is poured from the upstream of the protein purification unit 11 in the system shown in Fig. 1, the LRV only in the virus removal filter 12 cannot be measured accurately.

(Comparative Example 5)

In a manner similar to that of Comparative Example 4 except that a 0.5 mL column packed with a strong cation exchange chromatography carrier (Cellufine MAX GS, JNC) was used as the protein purification unit 11, the virus solution was poured into the protein purification system. The virus solution thus poured having a virus infectivity titer ($Log_{10} TCID_{50}$ (unit/mL)) of 7.741 flowed through the protein purification unit 11.

A virus infectivity titer in the collected permeate of the virus solution was measured. A log reduction value (LRV) in the protein purification unit 11 as calculated from the virus infectivity titer in the permeate of the virus solution and the virus infectivity titer in the virus solution before passing through the protein purification unit 11 was 0.25.

A virus infectivity titer in the collected permeate of the washing liquid was measured. A log reduction value (LRV) in the protein purification unit 11 as calculated from the virus infectivity titer in the permeate of the virus solution, the virus infectivity titer in the permeate of the washing liquid, and the virus infectivity titer in the virus solution before passing through the protein purification unit 11 was 0.21. The purification conditions and the purification results in Comparative Example 5 are shown in Fig. 8 and Fig. 9, respectively.

(Comparative Example 6)

In a manner similar to that of Comparative Example 4 except that a 0.5 mL column packed with a strong cation exchange chromatography carrier (Cellufine DexS-HbP, JNC) was used as the protein purification unit 11, the virus solution was poured into the protein purification system. The virus solution thus poured having a virus infectivity titer ($Log_{10} TCID_{50}$ (unit/mL)) of 7.738 flowed through the protein purification unit 11.

A virus infectivity titer in the collected permeate of the virus solution was measured. A log reduction value (LRV) in the protein purification unit 11 as calculated from the virus infectivity titer in the permeate of the virus solution and the virus infectivity titer in the virus solution before passing through the protein purification unit 11 was -0.39.

A virus infectivity titer in the collected permeate of the washing liquid was measured. A log reduction value (LRV) in the protein purification unit 11 as calculated from the virus infectivity titer in the permeate of the virus solution, the virus infectivity titer in the permeate of the washing liquid, and the virus infectivity titer in the virus solution before passing through the protein purification unit 11 was 0.38. The purification conditions and the purification results in Comparative Example 6 are shown in Fig. 8 and Fig. 9, respectively.

(Comparative Example 7)

In a manner similar to that of Comparative Example 4 except for the use of a virus solution containing 10% x-MuLV, the virus solution was poured into the protein purification system of Comparative Example 4. The virus solution thus poured had a virus infectivity titer ($Log_{10} TCID_{50}$ (unit/mL)) of 6.614.

A virus infectivity titer in the collected permeate of the virus solution was measured. A log reduction value (LRV) in the protein purification unit 11 as calculated from the virus infectivity titer in the permeate of the virus solution and the virus infectivity titer in the virus solution before passing through the protein purification unit 11 was 2.25.

The virus infectivity titer in the collected permeate of the washing liquid was measured. A log reduction value (LRV) in the protein purification unit 11 as calculated from the virus infectivity titer in the permeate of the virus solution, the virus infectivity titer in the permeate of the washing liquid, and the virus infectivity titer in the virus solution before passing through the protein purification unit 11 was 1.96. The purification conditions and the purification results in Comparative Example 7 are shown in Fig. 8 and Fig. 9, respectively.

(Comparative Example 8)

In a manner similar to that of Comparative Example 5 except for the use of a virus solution containing 10% x-MuLV, the virus solution was poured into the protein purification system of Comparative Example 4. The virus solution thus poured had a virus infectivity titer ($Log_{10} TCID_{50}$ (unit/mL)) of 6.239.

A virus infectivity titer in the collected permeate of the virus solution was measured. A log reduction value (LRV) in the protein purification unit 11 as calculated from the virus infectivity titer in the permeate of the virus solution and the virus infectivity titer in the virus solution before passing through the protein purification unit 11 was 0.13.

A virus infectivity titer in the collected permeate of the washing liquid was measured. A log reduction value (LRV) in the protein purification unit 11 as calculated from the virus infectivity titer in the permeate of the virus solution, the virus infectivity titer in the permeate of the washing liquid, and the virus infectivity titer in the virus solution before passing through the protein purification unit 11 was 0.21. The purification conditions and the purification results in Comparative Example 8 are shown in Fig. 8 and Fig. 9, respectively.

(Comparative Example 9)

In a manner similar to that of Comparative Example 6 except for the use of a virus solution containing 10% x-MuLV, the virus solution was poured into the protein purification system of Comparative Example 4. The virus solution thus poured had a virus infectivity titer ($Log_{10}$ $TCID_{50}$ (unit/mL)) of 6.368.

A virus infectivity titer in the collected permeate of the virus solution was measured. A log reduction value (LRV) in the protein purification unit 11 as calculated from the virus infectivity titer in the permeate of the virus solution and the virus infectivity titer in the virus solution before passing through the protein purification unit 11 was 0.44.

A virus infectivity titer in the collected permeate of the washing liquid was measured. A log reduction value (LRV) in the protein purification unit 11 as calculated from the virus infectivity titer in the permeate of the virus solution, the virus infectivity titer in the permeate of the washing liquid, and the virus infectivity titer in the virus solution before passing through the protein purification unit 11 was 0.38. The purification conditions and the purification results in Comparative Example 9 are shown in Fig. 8 and Fig. 9, respectively.

Reference Signs List

10: First channel
11: Protein purification unit
12: Virus removal filter
13: First pump
20: Second channel
21: Second pump
31: Protein concentration measuring instrument
32: Conductivity measuring instrument
33: In-line mixer

**Claims**

1. A viral clearance test method, comprising:

supplying a protein solution to a first channel provided with, upstream and downstream thereof, a protein purification unit and a virus removal filter, respectively, and pouring the protein solution into the protein purification unit at a first constant rate;
supplying a virus solution, at a second constant rate, to a second channel connected between the protein purification unit and the virus removal filter in the first channel and mixing the purified protein solution with the virus solution in the first channel;
pouring a mixture of the protein solution and the virus solution into the virus removal filter at a third constant rate; and
measuring a virus contained in a permeate of the mixture which has passed through the virus removal filter, wherein supposing that a represents the first constant rate, b represents the second constant rate, x represents a virus concentration in the mixture, and y represents a virus concentration in the virus solution, a, b, x, and y satisfy the following formula (1):

$$x/y = b/(a+b) \quad (1);$$

and

wherein supposing that C ($m^2$) represents a membrane area of the virus removal filter, $D_{min}$ represents a minimum value of b/(a+b), $D_{max}$ represents a maximum value of b/(a+b), $F_{min}$ (LMH) represents a minimum flux of the permeate in the virus removal filter, and **$F_{max}$** (LMH) represents a maximum flux of the permeate in the virus removal filter,

a minimum value $a_{min}$ (mL/min) of the first constant rate **a** is given by the following formula (2):

$$a_{min} = (1 - D_{max}) \ (1000/60) \ \times \ F_{min} \ \times \ C \quad (2),$$

a maximum value **$a_{max}$** (mL/min) of the first constant rate **a is** given by the following formula (3):

$$a_{max} = (1-D_{min}) \ (1000/60) \ \times \ F_{max} \ \times \ C \quad (3),$$

a minimum value $b_{min}$ (mL/min) of the second constant rate **b is** given by the following formula (4):

$$b_{min} = D_{min} \ (1000/60) \ \times \ F_{min} \ \times \ C \quad (4),$$

and
a maximum value $b_{max}$ (mL/min) of the second constant rate **b** is given by the following formula (5):

$$b_{max} = D_{max} \ (1000/60) \ \times \ F_{max} \ \times \ C \quad (5).$$

2. The method according to Claim 1, wherein the first channel is provided with a first pump for pouring the protein solution into the protein purification unit at the first constant rate.

3. The method according to Claim 1 or 2, wherein the second channel is provided with a second pump for pouring the virus solution at the second constant rate.

4. The method according to any one of Claims 1 to 3, wherein the protein solution is continuously poured into the protein purification unit.

5. The method according to any one of Claims 1 to 4, wherein the virus solution is continuously poured into the second channel.

6. The method according to any one of Claims 1 to 5, wherein the mixture is continuously poured into the virus removal filter.

7. The method according to any one of Claims 1 to 6, wherein a ratio of the second constant rate to a sum of the first constant rate and the second constant rate is 0.1% or more and 20% or less.

8. The method according to any one of Claims 1 to 7, wherein a virus infectivity titer ($Log_{10}$ $TCID_{50}$ (unit/mL)) in the mixture is 2 or more and 10 or less.

9. The method according to any one of Claims 1 to 8, wherein the virus removal filter has a membrane area of 0.0001 $m^2$ or more and 4 $m^2$ or less.

10. The method according to any one of Claims 1 to 9, wherein a flux of the permeate in the virus removal filter is 0.1 LMH or more and 500 LMH or less.

11. The method according to any one of Claims 1 to 10, further comprising:

after pausing the supply of the protein solution into the first channel, supplying the first channel with a washing liquid and pouring the washing liquid into the protein purification unit and the virus removal filter, and measuring a virus contained in a permeate of the washing liquid which has passed through the virus removal filter.

12. The method according to Claim 11, wherein time until the first channel is supplied with the washing liquid after the supply of the protein solution into the first channel is paused is 0 minutes or more and 24 hours or less.

**13.** The method according to any one of Claims 1 to 12, wherein the virus solution contains a protein.

**14.** The method according to any one of Claims 1 to 13, wherein the virus solution contains a protein the same as the protein contained in the protein solution.

**15.** The method according to Claim 13 or 14, wherein a concentration of the protein in the virus solution is equal to a concentration of the protein in the protein solution.

**16.** The method according to any one of Claims 1 to 15, further comprising:

comparing an amount of the virus contained in the mixture before passing through the virus removal filter with the amount of the virus contained in the permeate of the mixture which has passed through the virus removal filter.

**17.** The method according to any one of Claims 1 to 16, wherein the protein purification unit has a virus removal capacity.

**18.** The method according to Claim 17, wherein a log reduction value (LRV) in the protein purification unit 11 is 0 or more and 7 or less.

**Patentansprüche**

**1.** Virale-Clearance-Testverfahren, umfassend:

Zuführen einer Proteinlösung zu einem ersten Kanal, dem eine Proteinreinigungseinheit vorgeschaltet und ein Virusentfernungsfilter nachgeschaltet ist, und Gießen der Proteinlösung in die Proteinreinigungseinheit mit einer ersten konstanten Geschwindigkeit;
Zuführen einer Viruslösung mit einer zweiten konstanten Geschwindigkeit zu einem zweiten Kanal, der zwischen der Proteinreinigungseinheit und dem Virusentfernungsfilter im ersten Kanal angeschlossen ist, und Mischen der gereinigten Proteinlösung mit der Viruslösung im ersten Kanal;
Gießen eines Gemischs der Proteinlösung und der Viruslösung in das Virusentfernungsfilter mit einer dritten konstanten Geschwindigkeit; und
Messen des Virus, das in einem Permeat des Gemischs, das durch das Virusentfernungsfilter geflossen ist, enthalten ist,
wobei unter der Annahme, dass a für die erste konstante Geschwindigkeit steht, b für die zweite konstante Geschwindigkeit steht, x für die Viruskonzentration in dem Gemisch steht und **y** für die Viruskonzentration in der Viruslösung steht, a, b, x und **y** die folgende Formel (1) erfüllen:

$$x/y = b/(a+b) \qquad (1);$$

und
wobei unter der Annahme, dass C (m$^2$) für die Membranfläche des Virusentfernungsfilters steht, $D_{min}$ für das Minimum von b/(a+b) steht, $D_{max}$ für das Maximum von b/(a+b) steht, $F_{min}$ (LMH) für einen minimalen Fluss des Permeats in dem Virusentfernungsfilter steht und $F_{max}$ (LMH) für einen maximalen Fluss des Permeats in dem Virusentfernungsfilter steht,
das Minimum $a_{min}$ (ml/min) der ersten konstanten Geschwindigkeit a durch die folgende Formel (2) gegeben ist:

$$a_{min} = (1 - D_{max}) (1000/60) \times F_{min} \times C \qquad (2),$$

das Maximum $a_{max}$ (ml/min) der ersten konstanten Geschwindigkeit a durch die folgende Formel (3) gegeben ist:

$$a_{max} = (1 - D_{min}) (1000/60) \times F_{max} \times C \qquad (3),$$

das Minimum $b_{min}$ (ml/min) der zweiten konstanten Geschwindigkeit b durch die folgende Formel (4) gegeben ist:

$$b_{min} = D_{min} (1000/60) \times F_{min} \times C \qquad (4),$$

und

das Maximum $D_{max}$ (ml/min) der zweiten konstanten Geschwindigkeit b durch die folgende Formel (5) gegeben ist:

$$b_{max} = D_{max} (1000/60) \times F_{max} \times C \qquad (5).$$

2. Verfahren gemäß Anspruch 1, wobei der erste Kanal mit einer ersten Pumpe versehen ist, um die Proteinlösung mit der ersten konstanten Geschwindigkeit in die Proteinreinigungseinheit zu gießen.

3. Verfahren gemäß Anspruch 1 oder 2, wobei der zweite Kanal mit einer zweiten Pumpe versehen ist, um die Viruslösung mit der zweiten konstanten Geschwindigkeit zu gießen.

4. Verfahren gemäß einem der Ansprüche 1 bis 3, wobei die Proteinlösung kontinuierlich in die Proteinreinigungseinheit gegossen wird.

5. Verfahren gemäß einem der Ansprüche 1 bis 4, wobei die Viruslösung kontinuierlich in den zweiten Kanal gegossen wird.

6. Verfahren gemäß einem der Ansprüche 1 bis 5, wobei das Gemisch kontinuierlich in das Virusentfernungsfilter gegossen wird.

7. Verfahren gemäß einem der Ansprüche 1 bis 6, wobei das Verhältnis der zweiten konstanten Geschwindigkeit zu der Summe der ersten konstanten Geschwindigkeit und der zweiten konstanten Geschwindigkeit 0,1% oder mehr und 20% oder weniger beträgt.

8. Verfahren gemäß einem der Ansprüche 1 bis 7, wobei der Virustiter ($\log_{10}$ TCID$_{50}$ (Einheit/ml)) in dem Gemisch 2 oder mehr und 10 oder weniger beträgt.

9. Verfahren gemäß einem der Ansprüche 1 bis 8, wobei das Virusentfernungsfilter eine Membranfläche von 0,0001 m$^2$ oder mehr und 4 m$^2$ oder weniger aufweist.

10. Verfahren gemäß einem der Ansprüche 1 bis 9, wobei der Fluss des Permeats in dem Virusentfernungsfilter 0,1 **LMH** oder mehr und 500 **LMH** oder weniger beträgt.

11. Verfahren gemäß einem der Ansprüche 1 bis 10, weiterhin umfassend:

   nach einer Unterbrechung der Zufuhr der Proteinlösung zu dem ersten Kanal: Zuführen einer Waschflüssigkeit zu dem ersten Kanal und Gießen der Waschflüssigkeit in die Proteinreinigungseinheit und das Virusentfernungsfilter und
   Messen des Virus, das in einem Permeat der Waschflüssigkeit, die durch das Virusentfernungsfilter geflossen ist, enthalten ist.

12. Verfahren gemäß Anspruch 11, wobei die Zeit, bis dem ersten Kanal die Waschflüssigkeit zugeführt wird, nachdem die Zufuhr der Proteinlösung zu dem ersten Kanal unterbrochen wurde, 0 Minuten oder mehr und 24 Stunden oder weniger beträgt.

13. Verfahren gemäß einem der Ansprüche 1 bis 12, wobei die Viruslösung ein Protein enthält.

14. Verfahren gemäß einem der Ansprüche 1 bis 13, wobei die Viruslösung dasselbe Protein enthält wie die Proteinlösung.

15. Verfahren gemäß Anspruch 13 oder 14, wobei die Konzentration des Proteins in der Viruslösung gleich der Konzentration des Proteins in der Proteinlösung ist.

16. Verfahren gemäß einem der Ansprüche 1 bis 15, weiterhin umfassend:
   Vergleichen der Menge des in dem Gemisch enthaltenen Virus, bevor sie durch das Virusentfernungsfilter geflossen ist, mit der Menge des Virus, das in dem Permeat des Gemischs, das durch das Virusentfernungsfilter geflossen ist, enthalten ist.

**17.** Verfahren gemäß einem der Ansprüche 1 bis 16, wobei die Proteinreinigungseinheit eine Virusentfernungskapazität aufweist.

**18.** Verfahren gemäß Anspruch 17, wobei der logarithmische Reduktionswert (LRV) in der Proteinreinigungseinheit (11) 0 oder mehr und 7 oder weniger beträgt.


**Revendications**

**1.** Procédé de test de clairance virale, comprenant :

la fourniture d'une solution protéique dans un premier canal doté, en amont et en aval, d'une unité de purification de protéine et d'un filtre d'élimination de virus, respectivement, et l'injection de la solution protéique dans l'unité de purification de protéine à un premier débit constant ;

la fourniture d'une solution virale à un deuxième débit constant, à un deuxième canal relié entre l'unité de purification de protéine et le filtre d'élimination de virus dans le premier canal et le mélange de la solution protéique purifiée à la solution virale dans le premier canal ;

l'injection d'un mélange de la solution protéique et de la solution virale dans le filtre d'élimination de virus à un troisième débit constant ; et

la mesure d'un virus contenu dans un perméat du mélange qui a traversé le filtre d'élimination de virus,

dans lequel, en supposant que **a** représente le premier débit constant, **b** représente le deuxième débit constant, **x** représente une concentration virale dans le mélange et **y** représente une concentration virale dans la solution virale, a, **b, x** et **y** satisfont à la formule (1) suivante :

$$x/y = b/(a+b) \qquad (1) ;$$

et

dans lequel, en supposant que **C** ($m^2$) représente une zone de membrane du filtre d'élimination de virus, $D_{min}$ représente une valeur minimale de b/(a+b), $D_{max}$ représente une valeur maximale de b/(a+b), $F_{min}$ (LMH) représente un flux minimal du perméat dans le filtre d'élimination de virus et $F_{max}$ (LMH) représente un flux maximal du perméat dans le filtre d'élimination de virus,

une valeur minimale $a_{min}$ (ml/min) du premier débit constant **a** est donnée par la formule suivante (2) :

$$a_{min} = (1-D_{max}) \, (1000/60) \times F_{min} \times C \qquad (2),$$

une valeur maximale $a_{max}$ (ml/min) du premier débit constant **a** est donnée par la formule suivante (3) :

$$a_{max} = (1-D_{min}) \, (1000/60) \times F_{max} \times C \qquad (3),$$

une valeur minimale $b_{min}$ (ml/min) du deuxième débit constant **b** est donnée par la formule (4) suivante :

$$b_{min} = D_{min} \, (1000/60) \times F_{min} \times C \qquad (4),$$

et

une valeur maximale $b_{max}$ (ml/min) du deuxième débit constant **b** est donnée par la formule suivante (5) :

$$b_{max} = D_{max} \, (1000/60) \times F_{max} \times C \qquad (5).$$

**2.** Procédé selon la revendication 1, dans lequel le premier canal est doté d'une première pompe pour injecter la solution protéique dans l'unité de purification de protéine à un premier débit constant.

**3.** Procédé selon la revendication 1 ou la revendication 2, dans lequel le deuxième canal est doté d'une deuxième pompe pour injecter la solution virale à un deuxième débit constant.

**4.** Procédé selon l'une quelconque des revendications 1 à 3, dans lequel la solution protéique est injectée en continu dans l'unité de purification de protéine.

5. Procédé selon l'une quelconque des revendications 1 à 4, dans lequel la solution virale est injectée en continu dans le deuxième canal.

6. Procédé selon l'une quelconque des revendications 1 à 5, dans lequel le mélange est injecté en continu dans le filtre d'élimination de virus.

7. Procédé selon l'une quelconque des revendications 1 à 6, dans lequel le rapport entre le deuxième débit constant et une somme du premier débit constant et du deuxième débit est d'au moins 0,1 % et d'au plus 20 %.

8. Procédé selon l'une quelconque des revendications 1 à 7, dans lequel un titre d'infectiosité virale ($Log_{10}$ $TCID_{50}$ (unités/ml)) dans le mélange est de 2 ou plus et de 10 ou moins.

9. Procédé selon l'une quelconque des revendications 1 à 8, dans lequel le filtre d'élimination de virus présente une surface de membrane de 0,0001 $m^2$ ou plus et de 4 $m^2$ ou moins.

10. Procédé selon l'une quelconque des revendications 1 à 9, dans lequel un flux du perméat dans le filtre d'élimination de virus est de 0,1 LMH ou plus et de 500 LMH ou moins.

11. Procédé selon l'une quelconque des revendications 1 à 10, comprenant en outre :

   après avoir interrompu la fourniture de la solution protéique dans le premier canal, la fourniture au premier canal d'un liquide de lavage et l'injection du liquide de lavage dans l'unité de purification de protéine et le filtre d'élimination de virus, et
   la mesure d'un virus contenu dans un perméat du liquide de lavage qui a traversé le filtre d'élimination de virus.

12. Procédé selon la revendication 11, dans lequel le délai avant que le premier canal reçoive le liquide de lavage, après l'interruption de la fourniture de la solution protéique dans le premier canal, est de 0 minute ou plus et de 24 heures ou moins.

13. Procédé selon l'une quelconque des revendications 1 à 12, dans lequel la solution virale contient une protéine.

14. Procédé selon l'une quelconque des revendications 1 à 13, dans lequel la solution virale contient une protéine identique à la protéine contenue dans la solution protéique.

15. Procédé selon la revendication 13 ou la revendication 14, dans lequel une concentration en protéine dans la solution virale est égale à une concentration en protéine dans la solution protéique.

16. Procédé selon l'une quelconque des revendications 1 à 15, comprenant en outre :
la comparaison d'une quantité du virus contenu dans le mélange avant son passage à travers le filtre d'élimination de virus à la quantité du virus contenu dans le perméat du mélange qui a traversé le filtre d'élimination de virus.

17. Procédé selon l'une quelconque des revendications 1 à 16, dans lequel l'unité de purification de protéine présente une capacité d'élimination de virus.

18. Procédé selon la revendication 17, dans lequel une valeur de réduction logarithmique (LRV) dans l'unité de purification de protéine 11 est de 0 ou plus et de 7 ou moins.

Fig. 1

# Fig. 2

| | Virus | mAb concentration g/L | First pump flow rate mL/min | Protein solution purification unit | Column volume mL | Second pump flow rate mL/min | b/(a+b) % | Membrane area m2 |
|---|---|---|---|---|---|---|---|---|
| Ex. 1 | MVM | 5 | 0.225 | Mixed-mode AEX | 0.5 | 0.025 | 10 | 0.0003 |
| Ex. 2 | MVM | 5 | 0.225 | Grafted CEX | 0.5 | 0.025 | 10 | 0.0003 |
| Ex. 3 | MVM | 5 | 0.225 | Modified CEX | 0.5 | 0.025 | 10 | 0.0003 |
| Ex. 4 | x-MuLV | 5 | 0.225 | Mixed-mode AEX | 0.5 | 0.025 | 10 | 0.0003 |
| Ex. 5 | x-MuLV | 5 | 0.225 | Grafted CEX | 0.5 | 0.025 | 10 | 0.0003 |
| Ex. 6 | x-MuLV | 5 | 0.225 | Modified CEX | 0.5 | 0.025 | 10 | 0.0003 |

# Fig. 3

| | Virus | Permeate flux LMH | Virus titer $TCID_{50}/mL$ | LRV before process pause | Process pause minute | LRV after process pause |
|---|---|---|---|---|---|---|
| Ex. 1 | MVM | 50 | 6.884 | >5.56 | 35 | >5.19 |
| Ex. 2 | MVM | 50 | 6.813 | >5.50 | 35 | >5.13 |
| Ex. 3 | MVM | 50 | 6.875 | >5.56 | 35 | >5.19 |
| Ex. 4 | x–MuLV | 50 | 5.075 | >3.75 | 35 | >3.39 |
| Ex. 5 | x–MuLV | 50 | 4.939 | >3.62 | 35 | >3.26 |
| Ex. 6 | x–MuLV | 50 | 4.809 | >3.50 | 35 | >3.12 |

# Fig. 4

# Fig. 5

EP 4 317 460 B1

| | Virus | mAb concentration g/L | Pump flow rate mL/min | Protein solution purification unit | Membrane area m$^2$ |
|---|---|---|---|---|---|
| Comp. Ex. 1 | MVM | 5 | 0.025 | None | 0.0003 |
| Comp. Ex. 2 | MVM | 5 | 0.05 | None | 0.0003 |
| Comp. Ex. 3 | MVM | 5 | 0.1 | None | 0.0003 |

# Fig. 6

| | Virus | Permeate flux LMH | Virus titer $TCID_{50}/mL$ | LRV before process pause | Process pause minute | LRV after process pause |
|---|---|---|---|---|---|---|
| Comp. Ex. 1 | MVM | 5 | 6.13 | >5.27 | 35 | >5.13 |
| Comp. Ex. 2 | MVM | 10 | 6.25 | >5.40 | 35 | >5.24 |
| Comp. Ex. 3 | MVM | 20 | 6.44 | >5.59 | 35 | >5.43 |

# Fig. 7

# Fig. 8

EP 4 317 460 B1

|  | Virus | mAb concentration g/L | Pump flow rate mL/min | Protein solution purification unit | Column volume mL |
|---|---|---|---|---|---|
| Comp. Ex. 4 | MVM | 5 | 0.25 | Mixed-mode AEX | 0.5 |
| Comp. Ex. 5 | MVM | 5 | 0.25 | Grafted CEX | 0.5 |
| Comp. Ex. 6 | MVM | 5 | 0.25 | Modified CEX | 0.5 |
| Comp. Ex. 7 | x-MuLV | 5 | 0.25 | Mixed-mode AEX | 0.5 |
| Comp. Ex. 8 | x-MuLV | 5 | 0.25 | Grafted CEX | 0.5 |
| Comp. Ex. 9 | x-MuLV | 5 | 0.25 | Modified CEX | 0.5 |

# Fig. 9

EP 4 317 460 B1

| | Virus | Virus titer $TCID_{50}/mL$ | LRV before process pause | Process pause minute | LRV after process pause |
|---|---|---|---|---|---|
| Comp. Ex. 4 | MVM | 7.741 | 3.94 | nd | 1.96 |
| Comp. Ex. 5 | MVM | 7.741 | 0.25 | nd | 0.21 |
| Comp. Ex. 6 | MVM | 7.738 | −0.39 | nd | 0.38 |
| Comp. Ex. 7 | x−MuLV | 6.614 | 2.25 | nd | 1.96 |
| Comp. Ex. 8 | x−MuLV | 6.239 | 0.13 | nd | 0.21 |
| Comp. Ex. 9 | x−MuLV | 6.368 | 0.44 | nd | 0.38 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022072899 A1
- WO 20220407 A1
- WO 2014080676 A1
- US 20120088228
- JP 4024041 B

### Non-patent literature cited in the description

- Qualification of a novel inline spiking method for virus filter validation. **HERBERT LUTZ et al.** BIOTECH-NOLOGY PROGRESS. AMERICAN CHEMICAL SOCIETY, 27 September 2010, vol. 27, 121-128
- **BOHONAK DAVID M. et al.** Adapting virus filtration to enable intensified and continuous monoclonal antibody processing. *BIOTECHNOLOGY PROGRESS*, 11 December 2020, vol. 37 (2)
- Novel spiking methods developed for anion exchange chromatography operating in a continuous process. **YING LI et al.** BIOTECHNOLOGY AND BIOENGINEERING. JOHN WILEY, 30 July 2020, vol. 117, 3379-3389
- *Viral Safety - Practical Solutions for Risk Control*, 01 June 2018, 1-39, https://www.pall.com/content/dam/pall/biopharm/lit-library/gated/special/19-07511-USD3364-Virus-Safety-Practical-Solutions-Risk-Control-BOOK-EN.PDF
- **RAPHAEL WOLFISBERG et al.** *Journal of Virology*, 2016
- **BEATRIZ MAROTO et al.** *JOURNAL OF VIROLOGY*, 2004
- Virus Experiments, 22-23
- **V. HUTORNOJS**. *Env, Exp.*, 2012
- **ANTHONY M. D'ABRAMO JR. et al.** *Virology*, 2005
- **JOSHUA C GRIEGER et al.** *Molecular Therapy*, 2015
- **PAVEL PLEVKA et al.** *JOURNAL OF VIROLOGY*, 2011
- **PETER TATTERSALL et al.** *JOURNAL OF VIROLOGY*, 1976